# EUROPEAN PATENT SPECIFICATION

(11) **EP 4 279 108 B1**
(45) Date of publication and mention of the grant of the patent: **31.12.2025**
(21) Application number: 22174137.4
(22) Date of filing: 18.05.2022
(51) Int. Cl.: A61M 16/06

(54) **MINIMUM CONTACT RESPIRATORY NASAL MASK**
NASALE ATEMMASKE MIT MINIMALEM KONTAKT
MASQUE NASAL RESPIRATOIRE À CONTACT MINIMAL

(43) Date of publication of application: 22.11.2023
(73) Proprietor: Air Liquide Medical Systems, 92182 Antony Cedex (FR)
(72) Inventor: ALBERICI, Luca, 25073 Bovezzo (IT); SANDONI, Giuseppe, 25073 Bovezzo (IT); RUOCCO, Alessandra, 25073 Bovezzo (IT)
(74) Representative: Air Liquide

(56) References cited:
- EP-A1- 3 305 354
- EP-A2- 0 747 078
- WO-A1-2017/042717
- WO-A1-2020/208523
- US-A1- 2016 151 596
- US-A1- 2020 197 649

## Description

The invention concerns a respiratory nasal mask comprising a mask body and a flexible nasal cushion detachably coupled or fixed together, that is useable in the treatment of respiratory conditions or diseases affecting adults, children, toddlers, infants or newborns, such as obstructive sleep apnea (OSA).

Respiratory masks are commonly used for delivering a flow of breathable gas, such as air under pressure, for, or to assist in, patient respiration.

A mask assembly typically comprises a rigid or semi-rigid hollow shell or body, usually made of polymer, and further comprising a soft face-contacting cushion, commonly called a flexible cushion, that comes into contact with the patient's face and conforms to the various facial contours of the patient face for receiving the patient's nose and/or mouth so that the patient can inhale the gas comprised into a breathing chamber that is defined by the hollow shell or body and/or the flexible cushion.

The flexible cushion is usually made of soft, resilient elastomeric material, such as soft silicone or similar material.

A gas supply line delivers a respiratory gas, such as air under pressure, to the mask, namely the breathing chamber. An example of such a mask is given by EP-A-462701.

A headgear, comprising flexible straps or similar structures, is usually attached to the mask for correctly positioning, holding and/or securing the mask on the head of a patient. The headgear can be adjusted to pull the mask against the face with sufficient force to achieve a gas tight seal between the mask and the wearer's face as taught by EP-A-462701, EP-A-874667, EP-A-1972357 or WO-A-00/57942.

Other types of masks comprising a front shroud adapted to attach the headgear are disclosed by EP-A-2708258 or US-A-2007/0044804.

Masks receiving only the patient's nose are called a nasal mask, whereas those receiving the patient's nose and mouth are called facial or oro-nasal masks. Such masks are commonly used for treating respiratory conditions or diseases affecting adults or children, including some toddlers, infants or newborns, such as obstructive sleep apnea (OSA). For instance, the treatment of OSA or the like, for being efficient, requires that the patient uses the nasal mask overnight, namely when he/she sleeps.

Despite the fact that a lot of different architectures or masks have been proposed in the prior, especially for treating OSA or the like, problems or drawbacks still exist with existing nasal masks.

Thus, one important problem is related to the discomfort of use for the patient. Various causes of discomfort exist, such as gas leaks due to a wrong assembling of the mask components, a poor positioning of the mask of the patient's face, loose settings of the headgear, poor tightness of the cushion...

Gas leaks due to wrong assembling of the mask components can arise when the complexity of the mask is excessive, knowing that a patient has to disassemble and then re-assemble the components of the mask on regular basis, for instance every morning for cleaning them up.

Further, gas leaks due to a poor positioning of the mask can appear when the mask is too voluminous and hence difficult to be well-positioned by a user on his/her face, and/or to insufficient tightness of the cushion due to a poor contacting between the mask and patient's facial areas, especially for too heavy masks, that can be aggravated by loose settings of the headgear.

One can easily understand that those gas leaks and related discomfort for the patient can lead to a poor or inefficient treatment, including to an abandonment of their treatment by some patients.

Further, WO-A-2020/208523, WO-A-2017/042717, EP-A-3305354, US-A-2016/151596 and EP-A-747078 disclose different kinds of masks, including masks according to the preamble of Claim 1.

In this context, a goal of the present invention is to provide an improved nasal mask having a straightforward architecture for the user, i.e. a patient, and leading to an efficient tightness, thereby avoiding or minimizing the risks of incorrect assembling of different components, gas leaks and related discomfort for the patient. In particular, the nasal mask should be easy to assemble and/or to disassemble, in particular for cleaning operations after use or for replacing the cushion when worn out.

In other words, the goal of the present invention is to provide an improved respiratory nasal mask architecture that is compact, simple to assemble/disassemble, light and comfortable to wear, thanks to an improved architecture, thereby ensuring efficient positioning and securing of the mask on the patient's face, good comfort of use for the patients and an efficient gas tightness, i.e. seal, preventing the escape of gas that may result from a poor coupling of the components of the mask and/or from a poor contacting between the cushion and the facial areas of the patient.

A solution of the present invention concerns a respiratory nasal mask comprising:
- a mask body comprising :
   - a front section comprising a front opening and
   - a tubular rear section comprising a first inner passage and a rear opening, said rear opening being in fluid communication with the front opening via the first inner passage, and further comprising first fastening means and
- a hollow flexible cushion comprising :
   . a tubular front part comprising a front aperture and second fastening means, and
   . a supple rear part comprising a rear breathing opening for allowing, in use, gas exchanges with nostrils of a user, e.g. a patient, said rear breathing opening being in fluid communication with said front aperture, said supple rear part comprising peripheral edge regions surrounding the rear breathing opening that are configured to, in use, come into contact and provide a gas sealing with areas located immediately around the nostril edges of the user (P) comprising an upper lip area (ULA) located immediately under the nostrils, two curved alae (CA) on both sides of the nostrils and a tip region (TR) of the nose,
and wherein :
- the tubular rear section of the mask body is coaxially (axis XX) inserted into the tubular front part of the flexible cushion, and
- the first fastening means of the tubular rear section of the mask body cooperates with the second fastening means of the tubular front part of the flexible cushion for detachably fixing the hollow flexible cushion directly to the mask body.

Further, according to the invention, the rear surface of the front section of the mask body carries a collar structure projecting away from said rear surface and comprising a second inner passage in fluid communication with the front opening and with the first inner passage of the tubular rear section.

The invention is defined in the appended independent claim 1. Preferred embodiments are matter of the dependent claims.

The respiratory nasal mask according to the present invention can further comprise one or more of the following features.

### Mask body

The mask body of the nasal mask according to the present invention can further comprise one or more of the following additional features:
- the front section of the mask body comprises two lateral arms projecting laterally on each side of said mask body, i.e. on the left and right sides of the mask body, and in opposite directions.
- the two lateral arms are made one piece with the mask body.
- the two lateral arms and the mask body are made of polymer, preferably a rigid or semi-rigid polymer.
- the rigid or semi-rigid polymer, i.e. a plastic material, is polycarbonate (PC), polypropylene (PP), Nylon^{®}, or the like, for instance PC.
- the two lateral arms have an elongated shape, such as a band or ribbon shape. Other shapes are of course also possible.
- the lateral arms have each a length of about 3 to 10 cm, preferably of about 3 to 6 cm.
- each lateral arm comprise a free end with headgear connecting structures for attaching a headgear thereto.
- the headgear connecting structures comprise one or several slots, holes, hooks or the like.
- the front section of the mask body comprises a front wall traversed by the front opening, preferably a curved front wall.
- the front wall constitutes a body structure having a length (L) of between 40 and 70 mm and a width (W) of between 30 and 50 mm.
- the front wall has a thickness of between 2 and 15 mm.
- the front wall is flat.
- the front wall is slightly foldable, i.e. can be slightly bent, especially when tension forces are applied by the headgear connected to the two arms.
- the front wall comprises two opposite tips, the two lateral arms being attached to said two opposite tips.
- the front opening is arranged at the center of the front wall, namely is a central opening.
- the front opening has a circular shape.
- the front opening has a diameter of between 15 and 25 mm, preferably of between 17 and 22 mm.
- the collar structure projects axially (XX).
- the tubular rear section has an oval contour, i.e. an oval outer perimeter. "Oval" means strictly oval or ellipsoidal.
- the tubular rear section and the collar structure are coaxially (XX) arranged.
- the tubular rear section and the collar structure are spaced by a spacing of several millimeters.
- the tubular rear section is attached to the rear surface of the front wall of the mask body, preferably formed in one piece, e.g. molded in one piece.
- the tubular rear section, the collar structure and the front wall of the mask body are formed in one piece, e.g. molded in one piece.
- the venting orifices are in fluid communication with the inner breathing chamber of the flexible nasal cushion.
- the venting orifices are arranged in groups or arrays comprising each several venting orifices.
- groups or arrays of venting orifices are arranged so as to sandwich the front opening of the mask body, i.e. arranged to the left and to right of the front opening.
- the venting orifices are configured for venting to the atmosphere at least a part of the CO₂-contiaining gaseous flow expired by the user during the expiratory phases.
- the venting orifices have a cylindrical or tronconical shape, preferably their section is greater on the rear surface of the mask body than on the front surface of said mask body.
- the front opening and the collar structure of the mask body, and the front aperture of the flexible cushion are coaxially-arranged (XX).

### Cushion

The cushion of the nasal mask according to the present invention can further comprise one or more of the following additional features:
- it comprises an inner breathing chamber, i.e. an inner room or chamber for the gases, i.e. gas delivered to the user, such as a patient, during inspiratory phases and/or CO₂-enriched gases exhaled by the user during expiratory phases.
- it is sized and designed for not covering the regions of the nose of the user situated above his/her nose tip or tip region (TR), when the mask is worn by said user, especially the dorsum, i.e. cartilaginous and/or bony dorsum.
- it is made of a flexible material, i.e. a supple and soft or semi-soft material.
- it is made of silicone or the like.
- the inner breathing chamber of the cushion comprises the rear opening.
- the rear opening is in fluid communication with the inner breathing chamber.
- the front aperture or front opening is in fluid communication with the inner breathing chamber of the cushion.
- the front aperture has an oval shape, i.e. an oval section. "Oval" means strictly oval or ellipsoidal.
- the front aperture of the cushion has an oval shape that matches the oval contour of the tubular rear section of the mask body.
- the dimensions of the oval shape of the front aperture of the cushion are approximatively equal to the dimensions of the oval contour of the tubular rear section of the mask body so that the tubular rear section can be tightly inserted and secured into the front aperture of the cushion.
- the front aperture of the cushion is delimited by an inner border that surrounds the tubular rear section of the mask body, when the tubular rear section of the mask body is inserted into the tubular front part of the flexible cushion.
- the rear opening of the cushion has (almost or quasi) rectangular or trapezoidal shape.
- the peripheral edge regions surrounding the rear opening of the flexible cushion comprise a peripheral thin wall, such as a membrane or the like, constituting a sealing, preferably a supple sealing skirt, around said rear opening so as to ensure an efficient gas tightness while in contact with the user's face, when the latter wears the mask, preferably all along the outer periphery of said rear opening.
- the peripheral thin wall of the peripheral edge regions of the cushion forming the sealing comes into contact and provide a gas sealing with the areas located immediately around the nostril edges of the use, namely the upper lip area (ULA), the two curved alae (CA), and the tip region (TR) of the nose.
- the rear surface of the flexible cushion is curved, in particular for matching, in use, the two curved alae (CA) of the nose of the user.
- the peripheral edge regions surrounding the rear opening of the flexible nasal cushion form a curved (quasi) contacting surface, preferably a triangular or trapezoidal curved contacting surface.
- the curved (quasi) contacting surface surrounding the rear opening comes into contact, in use, i.e. when the user wears the mask, with the areas (i.e. user's facial areas) located immediately around the nostril edges of the user thereby providing an efficient gas sealing.
- the rear opening having a (almost or quasi) rectangular or trapezoidal shape is arranged in the curved (quasi) triangular contacting surface located around the rear breathing opening, preferably in the center of said curved (quasi) triangular contacting surface.
- the peripheral edge regions of the rear opening of the flexible cushion comprises a base border, two lateral panels or wings, and a top border.
- the width of the top border is larger/greater than the width of the base border, for instance the width of the top border is of between 17 and 25 mm, whereas the width W2 of the base border is of between 10 and 20 mm.
- the inner breathing chamber of the cushion is in fluid communication with the first inner passage of the tubular rear section of the mask body so that gas/gases can travel from the first inner passage of the tubular rear section of the mask body to the inner breathing chamber of the cushion, and vice versa, i.e. in both ways.
- the peripheral thin wall forming the sealing skirt around the rear breathing opening is molded in one piece with the rest of the cushion.
- a support-element is arranged in the rear opening.
- the support-element has an arch-shape or the like that projects into the cushion.
- the support-element has a "U" or "V"-shape or similar.
- the support-element forms a bridge between the upper and lower edges of the rear opening.
- when the mask is worn, the support-element is in contact with the outer region of the nose of the patient separating the nostrils so as to constitute a support or the like prohibiting a collapse, sagging or similar of the thin regions of the cushion, namely the peripheral edge regions of the rear opening of the flexible cushion, in particular the base and top borders.
- the peripheral edge regions of the rear opening of the flexible cushion are thinner than the tubular front part regions of the flexible cushion.
- the thickness of the wall of the flexible cushion in the peripheral edge regions of the rear opening is less than 1mm, preferably of between 0.30 and 0.6 mm.
- the thickness of the wall of the flexible cushion in the tubular front part regions of the flexible cushion is comprised between 1 and 5 mm, preferably of at least 2 mm.
- the flexibility of the cushion is greater in the peripheral edge regions of the rear opening than in the tubular front part regions of said flexible cushion.

### Connecting structures

The mask body and the cushion of the nasal mask according to the present invention comprise connecting structures for attaching the cushion directly to the the mask body, in a detachable manner so that the user can disassemble them for cleaning operations for instance.

Attaching "directly" the cushion to the mask body means that no intermediary piece or element, such as an annular ring or the like, is used for attaching them together.

Said connecting structures comprise the first fastening means of the tubular rear section of the mask body that cooperate with the second fastening means of the tubular front part of the flexible cushion for detachably fixing the hollow flexible cushion directly to the mask body, without the need of any intermediary member, such as a ring or similar.

Those connecting structures comprise can further comprise one or more of the following additional features:
- the first fastening means of the mask body comprise an annular shoulder or flange projecting outwardly, preferably radially.
- the annular shoulder or flange is arranged on the tubular rear section of the mask body, preferably on the outer peripheral wall of the tubular rear section, i.e. around its periphery.
- the annular shoulder or flange is arranged in the region of the rear opening of the tubular rear section of the mask body, i.e. close to said rear opening.
- the second fastening means of the flexible cushion comprise an annular groove arranged in the inner peripheral wall of the tubular front part of the flexible cushion.
- the annular shoulder or flange of the mask body is lodged and retained into the annular groove of the flexible cushion, when the hollow flexible cushion is attached to the mask body.
- the annular groove of the flexible cushion is laterally delimited by a first side wall and a second side wall facing each other.
- the annular shoulder or flange is sandwiched between said first side wall and second side wall, when said annular shoulder or flange is lodged into the annular groove of the flexible cushion.
- the said first side wall and second side wall constitute abutments that limit or block the course or motion of the annular shoulder or flange of the mask body into the annular groove of the flexible cushion.
- the first fastening means of the mask body cooperate with the second fastening means of the flexible cushion for further ensuring a gas tightness, in particular between the annular shoulder or flange and the annular groove, when said annular shoulder or flange of the mask body is lodged and retained into the annular groove of the flexible cushion, thereby avoiding gas leaks or similar.

### Hollow gas connector and flexible hose

A flexible hose can be fluidly connected to the front opening of the mask body of the nasal mask according to the present invention, by means of a hollow connector arranged at a proximal free end of said flexible hose.

The hollow connector can comprise one or more of the following additional features:
- it is a straight connector, i.e. tubular shape, preferably having a cylindrical shape.
- it comprise an inner passage for the gas, i.e. a lumen.
- it is inserted into and held in position in the mask body, through the front opening of the front section of the mask body.
- it is detachably fixed to the mask body.
- it is rotatable with respect to the mask body.
- it is configured for connecting a flexible hose thereto, i.e. a gas line or the like. In other words, the tubular connector is adapted for receiving a flexible conduct or hose that feeds a respiratory gas under pressure to the respiratory mask. The gas is fed into the flexible gas conduct or hose by a gas source, such as a medical ventilator, a medical gas-delivery device or the like.
- it is in fluid communication with the breathing chamber of the cushion thereby allowing gas to circulate from the hollow connector to the cushion or in the opposite way.
- it is inserted into the collar structure through the front opening of the mask body and secured therein, i.e. firmly hold therein, for instance by a snap-fit connection, a press-fit connection or others.
- it comprises a proximal end that is secured into the collar structure of the front section of the mask body, in particular inserted through the front opening.
- the proximal end of the hollow connector has a cylindrical or tronconical shape.
- according to another embodiment, the connector maybe a hollow curved or elbow connector, i.e. having a "L-shape" or the like.
- it is made of polymer material, e.g. plastic.

### Headgear

The nasal mask according to the present invention can further comprise a headgear comprising one or more of the following additional features:
- it is attached to the headgear connecting structures of the two lateral arms.
- it comprises straps that are used for positioning, maintaining and/or securing the mask in a desired position on the facial regions of the patient.
- the straps are made of polymer material or fabric material, or both.
- the straps comprise or are elongated flexible bands, ribbons or the like.
- the straps constitute a tridimensional structure.
- the straps comprise Velcro^{®}-like portions for forming loops or the like and allowing attaching the headgear to the headgear connecting structures of the mask body.

### Respiratory assembly

The invention also concerns a respiratory assembly or installation for providing gas to a patient (P) comprising a gas delivery device, such as a medical ventilator, i.e. a respiratory assistance device or the like, and a nasal mask according to the present invention, wherein the gas delivery device is connected to the nasal mask by means of the flexible hose.

Such a respiratory assembly comprising a nasal mask according to the present invention is usable in the treatment of respiratory conditions or diseases affecting adults, children, toddlers, infants or newborns, such as obstructive sleep apnea (OSA).

A non-limitative embodiment of a respiratory nasal mask according to the present invention is shown in the enclosed Figures, among which:
- Figure 1 represents a side view of a patient equipped with a nasal mask according to one embodiment of the present invention,
- Figure 2 is a front view of Figure 1,
- Figure 3 is a exploded view of the nasal mask of Figures 1 & 2,
- Figures 4 is a rear view of the flexible cushion of the nasal mask of Figures 1 & 2,
- Figures 5 is a front view of the flexible cushion of Figure 4,
- Figure 6 is a front view of the mask body of the mask of Figures 1 & 2,
- Figure 7 is a "3/4" side view of the mask body of Figure 6,
- Figure 8 is a rear view of the mask body of Figures 6 & 7, and
- Figure 9 and10 are sectional views of the mask of Figures 1 & 2.

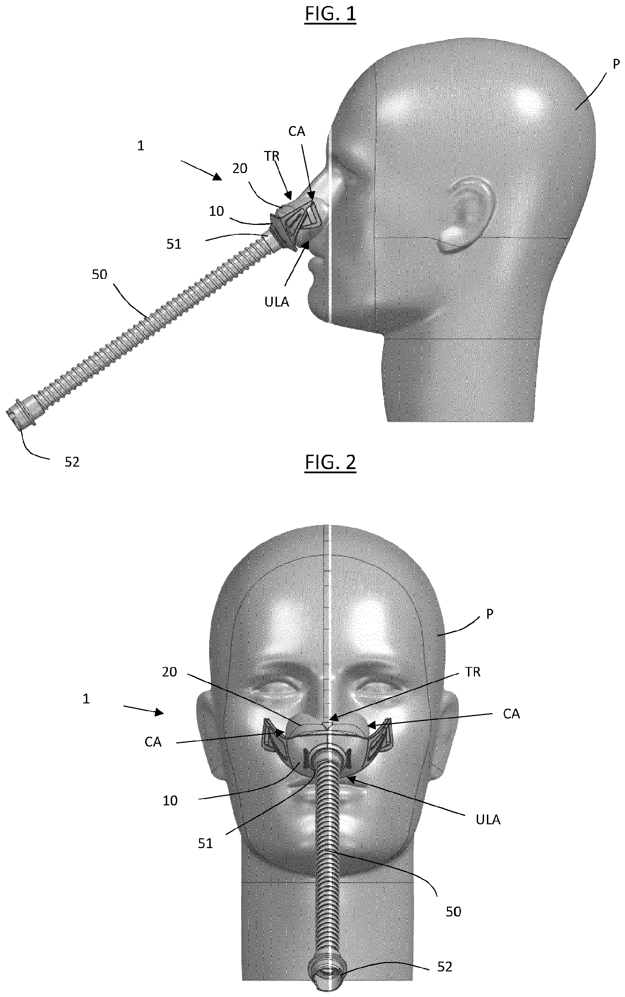

The present invention proposes a respiratory nasal mask 1 as shown in Figures 1-10 that illustrate an embodiment of a nasal mask 1 according to the present invention.

A nasal mask 1 according to the present invention comprises two main parts assembled or coupled together, namely a mask body 10 and a flexible cushion 20 attached together in a detachable manner so that they can be disassembled for instance for cleaning operations or for replacing a worn out cushion 20, and easily reassembled afterwards.

This mask 1 has a simple and light structure, and further facilitates the assembling/disassembling of the different components of the mask 1 by the users, namely the patients, especially during daily cleaning operations. Such a mask 1 further exhibits a good comfort as it is lighter and has a low risk of gas leaks as explained below.

As shown in Fig. 1&2, a respiratory gas, such as air, is provided by a flexible hose 50 fluidly and mechanically connected to the mask body 10 by means of a tubular connector 51 arranged at the proximal end of said flexible hose 50. The distal end of the flexible hose 50 comprise an additional tubular connector 52 for fluidly and mechanically connecting the flexible hose 50 to a medical ventilator (not shown) or a similar apparatus that provides the respiratory gas to the flexible hose 50.

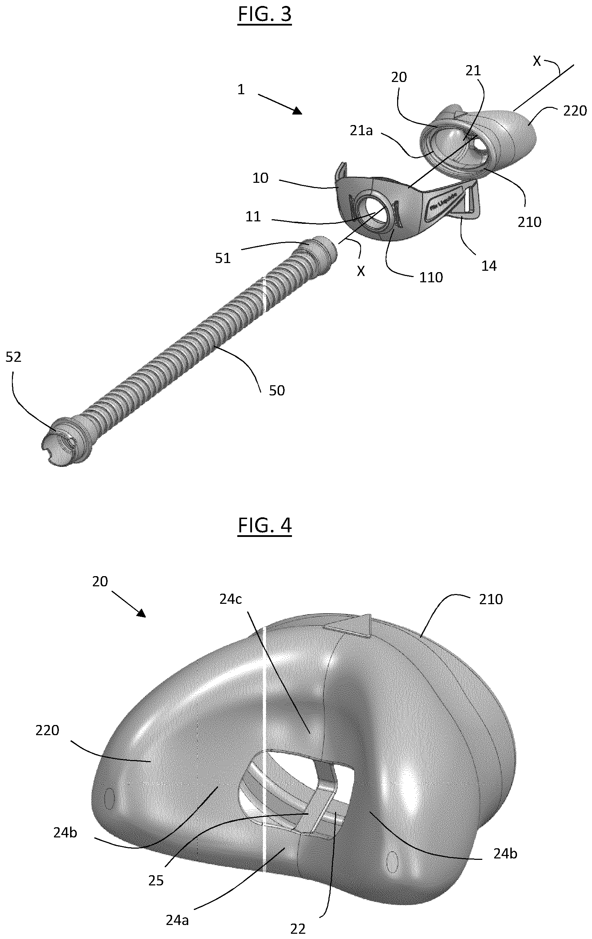

As shown in the Figures, the nasal mask 1 of the present invention comprises a mask body 10 comprising two lateral arms 14 projecting laterally, in opposite directions, i.e. one toward the right side of the mask body 10 and the other toward the left side of the mask body 10 as shown in Figures 3 and 6-8 for instance. The two lateral arms 14 are arranged around a front opening 11 traversing the mask body 10.

The two lateral arms 14 that project laterally may also constitute right and left cheek supports, when the mask is worn by a patient, as shown in Fig. 1&2.

Preferably, the two lateral arms 14 have elongated shapes, such as band or ribbon shapes, and/or can be slightly incurved to better match, if desired, some of the contours of the cushion 20. Each lateral arm 13 has for instance a length of about 3 to 10 cm, preferably of about 3 to 6 cm.

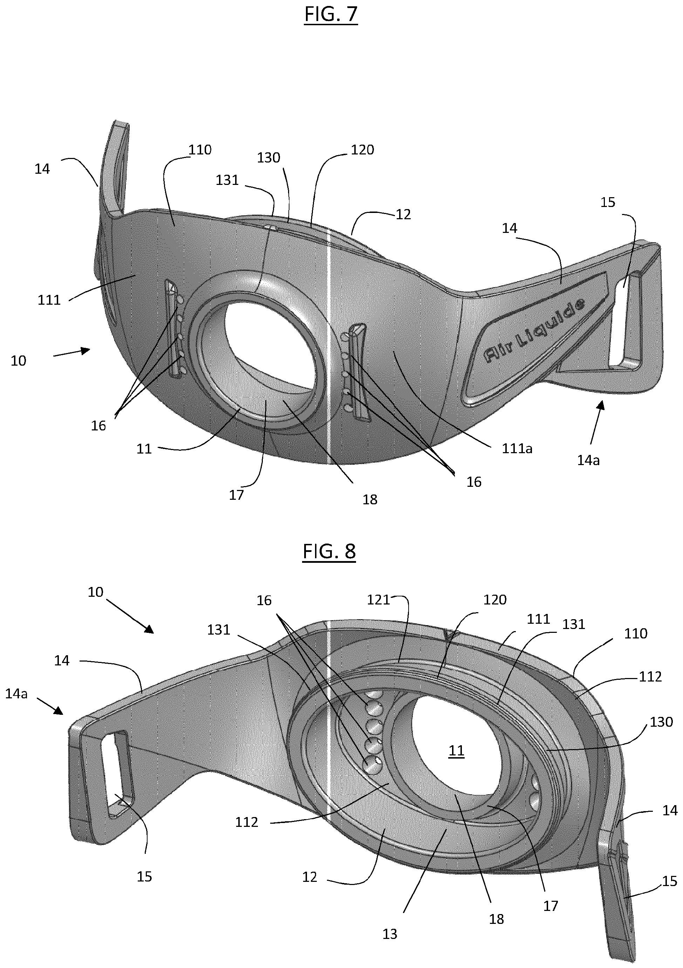

Further, as shown in Fig. 7&8, the lateral arms 14 comprise a free end 14a equipped with headgear connecting structures 15, such one or several slots, holes, hooks or the like. These headgear connecting structures 15 are designed for attaching a headgear thereto (not shown), typically the flexible straps of a headgear. A headgear is commonly used for maintaining and securing the mask 1 in a desired position on the head of the patient, as explained below. Preferably, the flexible straps of the headgear are made of polymer or fabric materials.

According to the present invention, the mask body 10 of the nasal mask 1, as shown in the Figures, comprises a front section 110 comprising a front opening 11 and a tubular rear section 120 comprising a first inner passage 13, i.e. a lumen or the like, and a rear opening 12. The rear opening 12 is in fluid communication with the front opening 11 via the first inner passage 13

The tubular rear section 120 further comprises first fastening means 130 for attaching the mask body 10 to the cushion 20, as explained below.

Further, the nasal mask 1 according to the present invention, as shown in the Figures, comprises a hollow flexible cushion 20 comprising a tubular front part 210 comprising a front aperture 21 and second fastening means 230 cooperating with the first fastening means 130, and further comprising a supple rear part 220 comprising a rear breathing opening 22 for allowing, in use, gas exchanges with the two nostrils of a user P.

The hollow flexible cushion 20 further comprises an inner breathing chamber 23 for containing the gas. The rear breathing opening 22 is in fluid communication with the front aperture 21, via the inner breathing chamber 23.

The supple rear part 220 comprises peripheral edge regions 24a-24c surrounding the rear breathing opening 22, as below detailed, that are configured to, in use, come into contact and provide a gas sealing with areas located immediately around the nostril edges of the user P comprising an upper lip area (ULA) located immediately under the nostrils, two curved alae (CA) on both sides of the nostrils and a tip region (TR) of the nose, thereby providing a minimum contact surface or the like with facial areas of the user.

The tubular rear section 120 of the mask body 10 is coaxially (i.e. axis XX) inserted into the tubular front part 210 of the flexible cushion 20.

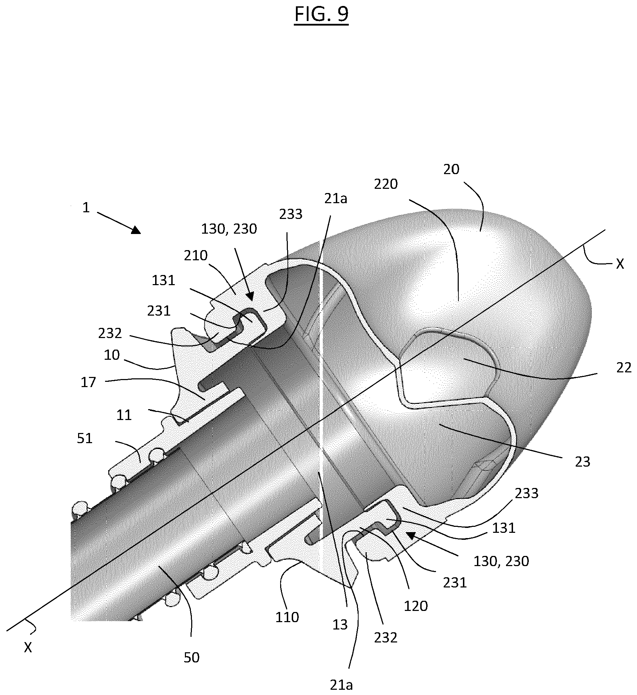

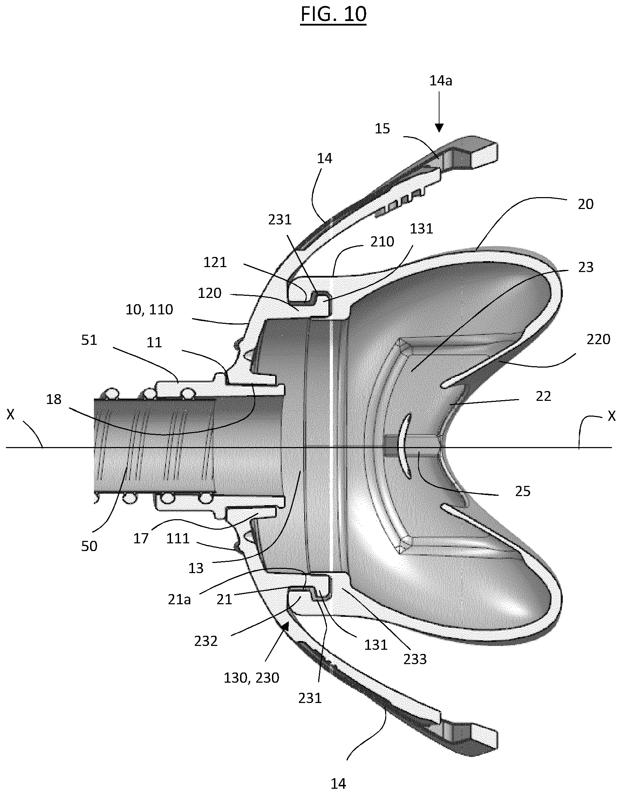

As below explained, the first fastening means 130 of the tubular rear section 120 of the mask body 10 cooperate with the second fastening means 230 of the tubular front part 210 of the flexible cushion 20 for detachably fixing the hollow flexible cushion 20 directly to the mask body 10 as shown in Fig. 9&10.

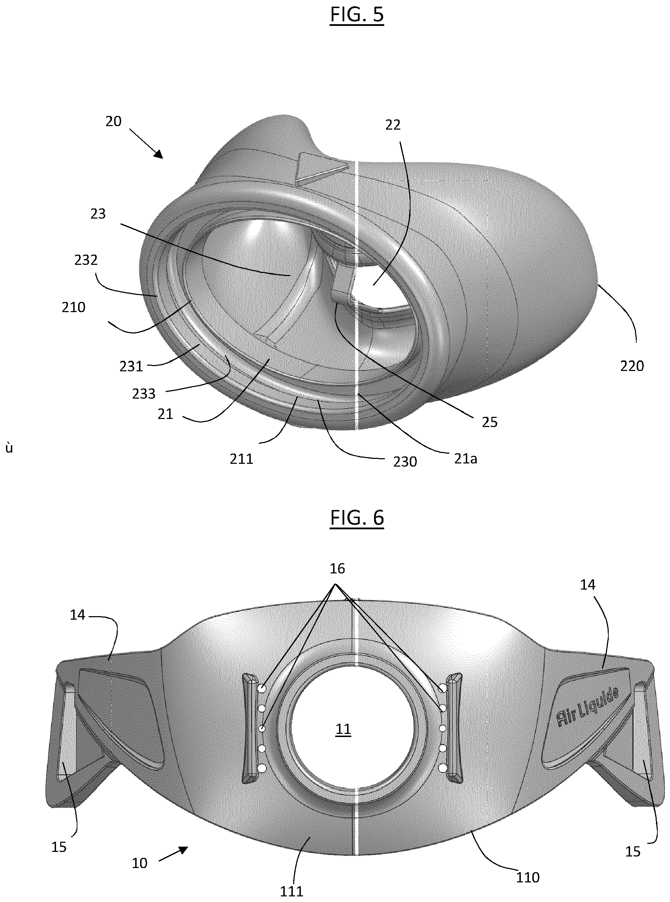

Further, the mask body 10 of the mask 1 of the present invention is shown in Fig. 6-8.

The mask body 10 comprises a front wall 111, preferably a curved and/or flat front wall 111, that is traversed by the front opening 11 that can have a circular shape and/or an inner diameter of about between 1 and 3 cm, for instance of between 15 and 25 mm. The front wall 111 of the front section 110 has a thickness of between 2 and 6 mm.

The mask body 10, in particular the front section 110 comprising the front opening 11 and the tubular rear section 120, and the two arms 14 are made of plastic material and molded in one piece, for instance by injection molding or any other suitable molding process. Typically, the plastic or polymer material can be PP, PC, PBT or nylon, or similar materials.

The front section 110 of the mask body 10 comprises two opposite surfaces 111a, 112, namely an outer front surface 111a (i.e. situated toward the outside) and a rear surface 112 facing the cushion 20 (i.e. situated toward the inside).

The rear surface 112 of the mask body 10 comprises a collar structure 17, i.e. a tubular structure, projecting away from said rear surface 112. The collar structure 17 is axially (XX) traversed by a second inner passage 18, i.e. a lumen, in fluid communication with the front opening 11 and with the first inner passage of the tubular rear section 120 of the mask body 10. The collar structure 17 projects axially (XX) toward the tubular rear section 120 and toward the cushion 20. Preferably, the collar structure 17 is cylindrical.

The tubular rear section 120 and the collar structure 17 are coaxially (XX) arranged and located at a distance from each other, i.e. spaced by a spacing of several millimeters.

The tubular rear section 120, the front section 110 and the collar structure 17 are preferably molded in one piece.

Further, the tubular rear section 120 has an oval contour 121, i.e. an oval outer perimeter. "Oval" means strictly oval or ellipsoidal.

As visible in Fig. 6-8, the front section 110 of the mask body 10 further comprises several venting orifices 16, i.e. traversing holes, slots or similar, traversing the front wall 110 and arranged around the front opening 11. They are configured for venting to the atmosphere at least a part of the CO₂-contiaining gaseous flow expired by the user P during his/her expiratory phases.

The venting orifices 16 are arranged in groups or arrays comprising each several venting orifices 16. For instance, in 2 arrays that sandwich the front opening 11 of the mask body 10, i.e. arranged to the left and to right of the front opening 11.

The venting orifices 16 are in fluid communication with the inner breathing chamber 23 of the flexible nasal cushion 20, via the tubular rear section 120.

Preferably, the venting orifices 16 traverse the front wall 111 and open on the rear surface 112 of the mask body 10 between the collar structure 17 and the tubular rear section 120 of the mask body 10 as visible in the Figures, namely in the spacing between the tubular rear section 120 and the collar structure 17 of the mask body 10.

The venting orifices 16 can have a cylindrical or tronconical shape, preferably their section is greater on the rear surface 112 than on the front surface 110 of said mask body, i.e. they have preferably a tronconical shape.

The front opening 11, the tubular rear section 120 and the collar structure 17 of the mask body 10, and the front aperture 21 of the flexible cushion 20 are coaxially-arranged (XX).

Further, the hollow flexible cushion 20 of the mask 1 of the present invention is better shown in Fig. 4 & 5.

The hollow flexible cushion 20 constitutes a tridimensional hollow flexible structure defining the inner breathing chamber 23. It further comprises a front aperture 21 and a rear opening 22 that fluidly communicate with the inner breathing chamber 23.

The cushion is preferably made of silicone or the like.

When the mask 1 is worn, the supple rear part 220 of the flexible cushion 20 comprises a contact zone located on its outer surface that comes into contact, in use, with the patient's face, namely regions located immediately around the nostrils of the patient P, in particular the upper lip area (ULA) located immediately under the nostrils, two curved alae (CA) on both sides of the nostrils and the tip region (TR) of the nose as shown in Fig. 1&2.

Said contact zone comprises peripheral edge regions 24a-24c surrounding the rear breathing opening 22 supple rear part 220 as shown in Fig. 4, i.e. regions located immediately around the rear breathing opening 22.

Said peripheral edge regions 24a-24c are configured to, in use, come into contact and provide a gas sealing surface with areas located immediately around the nostril edges of the user P, i.e. the upper lip area (ULA), the two curved alae (CA) and the region (TR) of the nose of the user P.

The cushion 20 of the nasal mask 1 of the present invention has a compact size and does not extend over the top of the nose, namely does not cover the dorsum regions, i.e. cartilaginous and/or bony dorsum, when the mask 1 is worn by a user P as shown in Fig. 1&2.

Such a compact cushion 20 provides several advantages such as it can easily adapted to more patients having various facial morphologies, since the prominence of their nose has no effect, when compared to traditional cushions where the top of the patient's nose has to be inserted far into the breathing chamber and hence can touch some internal parts.

Generally speaking, the compact cushion 20 of the nasal mask 1 of the present invention reduces and/or minimizes the contact with the skin of the user as it rests only on the areas located immediately around the entrances of the nostrils of said user. In other words, the compact cushion 20 of the invention can be sized and/or designed in taking into account only the width of the bottom of the nose of the user.

Furthermore, in order to provide efficient gas tightness (i.e. seal) and/or good comfort for the patient, the cushion wall in the peripheral edge regions 24a-24c surrounding the rear opening 22 of the supple rear part 220 of the cushion 20 is preferably designed or conceived as a thin membrane, i.e. a soft flexible membrane, that comes into contact with the patient's facial areas or regions, namely the upper lip area (ULA), the two curved alae (CA) and tip region (TR) as above stated, so as to better match his/her facial morphology.

Said flexible thin membrane forms a soft skirt or the like all along the edges of the rear opening 22 of the cushion 20. A unique membrane is preferably used; however, in alternative embodiments, several membranes may be superimposed.

As better shown in Fig. 4, the rear outer surface of the cushion 30 that comprises the peripheral edge regions 24a-24c surrounding the rear breathing opening 22, has a curved shape for better matching some nasal regions, in particular the two curved alae (CA) situated on both sides of the nostrils of the user P.

More precisely, said outer curved rear surface of the supple rear part 220 of the peripheral edge regions 24a-24c surrounding the rear breathing opening 22 of the flexible nasal cushion 20 comprises :
- a base or bottom border 24a that comes, in use, into contact with the upper lip area (ULA) of the user,
- two lateral panels or wings 24b that come, in use, into contact with the two curved alae (CA) on both sides of the nostrils of the user P, and
- a top border 24c that comes, in use, into contact with the tip region (TR) of the nose of the user P.

The width W1 of the top border 24c is greater than the width W2 of the base border 24a. For instance, W1 is comprised between 17 and 25 mm, whereas W2 is comprised between 10 and 20 mm.

In other words, the cushion 30 has a tridimensional form or shape that well-matches the contours of the patient's nasal regions (i.e. ULA, CA, TR), in particular thanks to its curved rear surface, thereby forming a contact zone constituting a gas seal, i.e. improved tightness.

Further, as shown in Fig. 4, the rear breathing opening 22 has a (quasi-) rectangular or similar (e.g. trapezoidal shape). It is arranged at the center of the curved rear surface including the peripheral edge regions 24a-24c surrounding the rear breathing opening 23 of the supple rear part 220 of the flexible nasal cushion 20.

In an embodiment, the supple rear part 220 of the flexible nasal cushion 20 comprises a support-element 25, such as a little band or the like that is arranged in the rear opening 22. It is preferably molded with the supple rear part 220 of the cushion 20.

As shown in Fig. 4, the extremities of the support-element 25 forms a bridge between the upper and lower edges of the rear opening 22. The support-element 25 has an arch-shape, for instance a "U" or "V"-shape, that slightly projects into the inner breathing chamber 23 of the cushion 20.

When the mask 1 is worn by the patient P, as shown in Fig. 1&2, the support-element 25 comes into contact with the outer region of the nose of the patient P separating the nostrils, namely the nasal columella, so as to constitute a support or the like prohibiting a collapse, sagging or similar of the thin regions of the supple rear part 220 of the cushion, namely the peripheral edge regions 24a-24c, in particular the base 24a and top 24c borders

Furthermore, the tubular front part 210 of the cushion 20 comprises a front aperture 21, i.e. a gas opening, having an oval shape, i.e. an oval section. "Oval" means strictly oval or ellipsoidal.

The oval shape of the front aperture 21 of the cushion 20 matches the oval contour of the tubular rear section 120 of the mask body 10, i.e. both have complementary oval forms. In other words, the dimensions of the oval shape of the front aperture 21 of the cushion 20 are approximately equal to the dimensions of the oval contour of the tubular rear section 120 of the mask body 10 so that the tubular rear section 120 can be tightly inserted and secured into the front aperture 21 of the cushion 20 as shown in Fig. 9&10.

Further, the front aperture 21 of the cushion 20 is delimited by an inner border 21a that surrounds the tubular rear section 120 of the mask body 10, when said tubular rear section 120 of the mask body 10 is inserted into the tubular front part 210 of the flexible cushion 20.

According to the present invention, for ensuring an efficient coupling of the cushion 20 directly to the mask body 10, the tubular front part 210 of the flexible cushion 20 further comprises second fastening means 230 that are configured for cooperating with the first fastening means 130 of the tubular rear section 120 of the mask body 10.

In other words, for ensuring a firm and tight attachment or, conversely, for allowing a detachment of the cushion 20 to the mask body 10 for cleaning operations or the like, it is provided suitable connecting means or structures cooperating together, namely the first and second fastening means 130, 230.

As shown in Fig. 9&10, the first fastening means 130 arranged on the tubular rear section 120 of the mask body 10 comprise an annular shoulder or flange 131 projecting outwardly, preferably radially.

Said annular shoulder or flange 131 is arranged on the outer peripheral wall of the tubular rear section 130, i.e. around its periphery, having an oval contour as above explained. The annular shoulder or flange is located in the region of the rear opening 12 of the mask body 10, i.e. close to said rear opening 12.

Further, the second fastening means 230 comprise an annular groove 231 arranged in the inner peripheral wall 211 of the tubular front part 210 of the flexible cushion 20 as shown in Fig. 5, 9 & 10.

Said annular groove 231 is laterally delimited by a first side wall 232 and a second side wall 233 facing each other.

When the hollow flexible cushion 20 is attached to the mask body 10, the annular shoulder or flange 131 of the mask body 10 is lodged and retained into the annular groove 231 of the flexible cushion 20, i.e. the annular shoulder or flange 131 is sandwiched between the first and second side walls 232, 233. The first and second side walls 232, 233 are linked, i.e. joined, by a bottom wall forming the bottom of the groove 231.

Actually, said first and second side walls 232, 233 constitute abutments or the like that limit or block the course or motion of the annular shoulder or flange 131 into the annular groove 231 of the flexible cushion 20.

Generally speaking, the compact nasal mask 1 of the present invention is light and comfortable to wear. Such a simple architecture leads to a reduction of the overall size and weight of the mask 1, allowing easy assembling and/or disassembling operations and efficient positioning and securing of the mask 1 on the patient's nasal regions, as well as a good tightness (i.e. seal) and an improved comfort of use for the patient.

The nasal mask 1 of the present invention can be used in a method for treatment of a respiratory disorder or condition affecting infant, toddler, child or adult patients, such as OSA or other respiratory troubles.

## Claims

1. Respiratory nasal mask (1) comprising:
- a mask body (10) comprising :
. a front section (110) comprising a front opening (11) and a rear surface (112),
. a tubular rear section (120) comprising a first inner passage (13) and a rear opening (12), said rear opening (12) being in fluid communication with the front opening (11) via the first inner passage (13), and further comprising first fastening means (130) and
- a hollow flexible cushion (20) comprising :
. a tubular front part (210) comprising a front aperture (21) and second fastening means (230), and
. a supple rear part (220) comprising a rear breathing opening (22) for allowing, in use, gas exchanges with nostrils of a user (P), said rear breathing opening (22) being in fluid communication with said front aperture (21), said supple rear part (220) comprising peripheral edge regions (24) surrounding the rear breathing opening (22) that are configured to, in use, come into contact and provide a gas sealing with areas located immediately around the nostril edges of the user (P) comprising an upper lip area (ULA) located immediately under the nostrils, two curved alae (CA) on both sides of the nostrils and a tip region (TR) of the nose,
and wherein :
- the rear surface (112) of the mask body (10) is facing the flexible cushion (20),
- the tubular rear section (120) of the mask body (10) is coaxially (XX) inserted into the tubular front part (210) of the flexible cushion (20), and
- the first fastening means (130) of the tubular rear section (120) of the mask body (10) cooperate with the second fastening means (230) of the tubular front part (210) of the flexible cushion (20) for detachably fixing the hollow flexible cushion (20) directly to the mask body (10),
**characterized in that** :
- the rear surface (112) of the front section (110) of the mask body (10) carries a collar structure (17) projecting away from said rear surface (112) toward the flexible cushion (20), and comprising a second inner passage (18) in fluid communication with the front opening (11) and with the first inner passage (13) of the tubular rear section (120), the tubular rear section (120) and the collar structure (17) being coaxially (XX) arranged and at a distance from each other, and
- the front section (110) of the mask body (10) further comprises several venting orifices (16) traversing the front wall (110), arranged around the front opening (11) and opening on the rear surface (112) of the mask body (10) in the spacing between the collar structure (17) and the tubular rear section (120) of the mask body (10).

2. Mask according to Claim 1, **characterized in that** the front section (110) of the mask body (10) further comprises two lateral arms (14) projecting laterally on each side of said mask body (10), preferably the two lateral arms (14) are made one piece with the mask body (10).

3. Mask according to Claim 1, **characterized in that** :
- the first fastening means (130) of the mask body (10) comprise an annular shoulder (131) projecting outwardly arranged on the tubular rear section (120) of the mask body (10),
- the second fastening means (230) of the flexible cushion (20) comprise an annular groove (231) arranged in the inner peripheral wall (211) of the tubular front part (210) of the flexible cushion (20), and
- the annular shoulder (131) of the mask body (10) is lodged and retained into the annular groove (231) of the flexible cushion (20), when the hollow flexible cushion (20) is attached to the mask body (10).

4. Mask according to Claim 3, **characterized in that** the annular groove (231) is laterally delimited by a first side wall (232) and a second side wall (233) facing each other, and when the hollow flexible cushion (20) is attached to the mask body (10) the annular shoulder (131) of the mask body (10) is lodged and retained into the annular groove (231) of the flexible cushion (20) so that the annular shoulder (131) is sandwiched between the first and second side walls (232, 233), said first and second side walls (232, 233) constituting abutments that limit or block the course or motion of the annular shoulder (131) into the annular groove (231) of the flexible cushion (20).

5. Mask according to Claim 1, **characterized in that** the tubular rear section (120), the collar structure (17) and the front section (110) of the mask body (10) are molded in one piece.

6. Mask according to Claim 1, **characterized in that** the annular groove (231) of the flexible cushion (20) is laterally delimited by a first side wall (232) and a second side wall (232) facing each other, the annular shoulder (131) being sandwiched between said first side wall (232) and second side wall (232), when said annular shoulder (131) is lodged into the annular groove (231) of the flexible cushion (20).

7. Mask according to Claim 1, **characterized in that** the hollow flexible cushion (20) comprises an inner breathing chamber (23), the front aperture (21) and the rear breathing opening (22) of the flexible cushion (20) being in fluid communication with said inner breathing chamber (23).

8. Mask according to Claim 1, **characterized in that** the collar structure (17) is configured for securing a tubular hollow connector (51) inserted into the second inner passage (18) through the front opening (11) of the front section (110).

9. Mask according to Claim 1, **characterized in that** :
- the tubular rear section (120) of the mask body (10) has an oval contour (121) and
- the front aperture (21) of the cushion (20) has an oval shape (212) that matches the oval contour (121) of the tubular rear section (120) of the mask body (10), when the tubular rear section (120) of the mask body (10) is inserted into the tubular front part (210) of the cushion (20), through the front aperture (21) of the cushion (21).

10. Mask according to Claim 1, **characterized in that** the supple rear part (220) of the flexible cushion (20) comprises peripheral edge regions (24a-24c) surrounding the rear breathing opening (22), said peripheral edge regions (24a-24c) comprise a peripheral thin wall, such as a membrane, forming a sealing around said rear opening (22), when the mask (1) is worn.

11. Mask according to Claim 1, **characterized in that** the cushion (20) is made of silicone.

12. Mask according to Claim 1, **characterized in that** a support-element (25) projecting into the cushion (20) is arranged in the rear opening (22).

13. Mask according to Claim 1, **characterized in that** a tubular hollow connector (51) is inserted into the collar structure (17) through the front opening (11) of the mask body (10) and secured therein, preferably the tubular hollow connector (51) is arranged on a flexible hose (50).

14. Installation for providing gas to a patient (P) comprising a medical ventilator and a respiratory nasal mask (1) according to any one of the preceding Claims, said medical ventilator being fluidly and mechanically connected to the respiratory nasal mask (1) by means of the flexible hose (50) comprising the tubular hollow connector (51).

## Patentansprüche

1. Nasenmaske (1) für die Atemwege, umfassend:
- einem Maskenkörper (10), der umfasst:
. einen vorderen Abschnitt (110) mit einer vorderen Öffnung (11) und einer Rückseite (112),
. einen röhrenförmigen hinteren Abschnitt (120) mit einem ersten inneren Durchgang (13) und einer hinteren Öffnung (12), wobei die hintere Öffnung (12) über den ersten inneren Durchgang (13) in Fluidverbindung mit der vorderen Öffnung (11) steht, und ferner mit einer ersten Befestigungsvorrichtung (130) und
- ein hohles flexibles Polster (20), das umfasst:
. einem röhrenförmigen Vorderteil (210) mit einer vorderen Öffnung (21) und zweiten Befestigungsmitteln (230) und
. einen geschmeidigen hinteren Teil (220) mit einer hinteren Atemöffnung (22), um im Gebrauch einen Gasaustausch mit den Nasenlöchern eines Benutzers (P) zu ermöglichen, wobei die hintere Atemöffnung (22) in Fluidverbindung mit der vorderen Öffnung (21) steht, wobei der geschmeidige hintere Teil (220) Randbereiche (24) aufweist, die die hintere Atemöffnung umgeben (22) umgeben, die so konfiguriert sind, dass sie bei Gebrauch mit Bereichen in Kontakt kommen und eine Gasabdichtung mit Bereichen bilden, die sich unmittelbar um die Nasenflügelränder des Benutzers (P) befinden, einschließlich eines Oberlippenbereichs (ULA), der sich unmittelbar unter den Nasenflügeln befindet, zwei gekrümmten Nasenflügeln (CA) auf beiden Seiten der Nasenflügel und einem Nasenspitzenbereich (TR),
und wobei:
- die Rückseite (112) des Maskenkörpers (10) dem flexiblen Polster (20) zugewandt ist,
- der röhrenförmige hintere Abschnitt (120) des Maskenkörpers (10) koaxial (XX) in den röhrenförmigen vorderen Teil (210) des flexiblen Polsters (20) eingeführt ist, und
- die ersten Befestigungsmittel (130) des röhrenförmigen hinteren Abschnitts (120) des Maskenkörpers (10) mit den zweiten Befestigungsmitteln (230) des röhrenförmigen vorderen Teils (210) des flexiblen Polsters (20) zusammenwirken, um das hohle flexible Polster (20) direkt am Maskenkörper (10) lösbar zu befestigen,
**dadurch gekennzeichnet, dass**:
- die Rückseite (112) des vorderen Abschnitts (110) des Maskenkörpers (10) eine Kragenstruktur (17) aufweist, die von der Rückseite (112) in Richtung des flexiblen Polsters (20) vorsteht und einen zweiten inneren Durchgang (18) umfasst, der in Fluidverbindung mit der vorderen Öffnung (11) und mit dem ersten inneren Durchgang (13) des röhrenförmigen hinteren Abschnitts (120) steht, wobei der röhrenförmige hintere Abschnitt (120) und die Kragenstruktur (17) koaxial (XX) und in einem Abstand voneinander angeordnet sind, und
- der vordere Abschnitt (110) des Maskenkörpers (10) umfasst ferner mehrere Entlüftungsöffnungen (16), die die Vorderwand (110) durchqueren, um die vordere Öffnung (11) herum angeordnet sind und sich auf der Rückseite (112) des Maskenkörpers (10) im Abstand zwischen der Kragenstruktur (17) und dem röhrenförmigen hinteren Abschnitt (120) des Maskenkörpers (10) öffnen.

2. Maske nach Anspruch 1, **dadurch gekennzeichnet, dass** der vordere Abschnitt (110) des Maskenkörpers (10) ferner zwei seitliche Arme (14) umfasst, die seitlich auf jeder Seite des Maskenkörpers (10) vorstehen, wobei die beiden seitlichen Arme (14) vorzugsweise einstückig mit dem Maskenkörper (10) ausgebildet sind.

3. Maske nach Anspruch 1, **dadurch gekennzeichnet, dass**:
- die ersten Befestigungsmittel (130) des Maskenkörpers (10) eine ringförmige, nach außen vorstehende Schulter (131) umfassen, die am röhrenförmigen hinteren Abschnitt (120) des Maskenkörpers (10) angeordnet ist,
- die zweite Befestigungsvorrichtung (230) des flexiblen Polsters (20) eine ringförmige Nut (231) umfasst, die in der inneren Umfangswand (211) des röhrenförmigen Vorderteils (210) des flexiblen Polsters (20) angeordnet ist, und
- die ringförmige Schulter (131) des Maskenkörpers (10) in der ringförmigen Nut (231) des flexiblen Polsters (20) sitzt und gehalten wird, wenn das hohle flexible Polster (20) am Maskenkörper (10) befestigt ist.

4. Maske nach Anspruch 3, **dadurch gekennzeichnet, dass** die ringförmige Nut (231) seitlich durch eine erste Seitenwand (232) und eine zweite Seitenwand (233) begrenzt ist, die einander gegenüberliegen, und wenn das hohle flexible Polster (20) am Maskenkörper (10) angebracht wird, die ringförmige Schulter (131) des Maskenkörpers (10) in die ringförmige Nut (231) des flexiblen Polsters (20) eingeklemmt und darin gehalten wird, so dass die ringförmige Schulter (131) zwischen der ersten und der zweiten Seitenwand (232, 233) eingeklemmt wird, wobei die erste und die zweite Seitenwand (232, 233) Anschläge bilden, die den Verlauf oder die Bewegung der ringförmigen Schulter (131) in die ringförmige Nut (231) des flexiblen Kissens (20) begrenzen oder blockieren.

5. Maske nach Anspruch 1, **dadurch gekennzeichnet, dass** der röhrenförmige hintere Abschnitt (120), die Kragenstruktur (17) und der vordere Abschnitt (110) des Maskenkörpers (10) einstückig geformt sind.

6. Maske nach Anspruch 1, **dadurch gekennzeichnet, dass** die ringförmige Nut (231) des flexiblen Polsters (20) seitlich durch eine erste Seitenwand (232) und eine zweite Seitenwand (232) begrenzt ist, die einander gegenüberliegen, wobei die ringförmige Schulter (131) zwischen der ersten Seitenwand (232) und der zweiten Seitenwand (232) angeordnet ist, wenn die ringförmige Schulter (131) in die ringförmige Nut (231) des flexiblen Polsters (20) eingepasst ist.

7. Maske nach Anspruch 1, **dadurch gekennzeichnet, dass** das hohle flexible Polster (20) eine innere Atemkammer (23) umfasst, wobei die vordere Öffnung (21) und die hintere Atemöffnung (22) des flexiblen Polsters (20) in Fluidverbindung mit der inneren Atemkammer (23) stehen.

8. Maske nach Anspruch 1, **dadurch gekennzeichnet, dass** die Kragenstruktur (17) so konfiguriert ist, dass sie einen röhrenförmigen hohlen Verbinder (51) sichert, der durch die vordere Öffnung (11) des vorderen Abschnitts (110) in den zweiten inneren Durchgang (18) eingeführt wird.

9. Maske nach Anspruch 1, **dadurch gekennzeichnet, dass**:
- der röhrenförmige hintere Abschnitt (120) des Maskenkörpers (10) eine ovale Kontur (121) aufweist und
- die vordere Öffnung (21) des Polsters (20) eine ovale Form (212) aufweist, die der ovalen Kontur (121) des röhrenförmigen hinteren Abschnitts (120) des Maskenkörpers (10) entspricht, wenn der röhrenförmige hintere Abschnitt (120) des Maskenkörpers (10) in den röhrenförmigen vorderen Teil (210) des Polsters (20) durch die vordere Öffnung (21) des Polsters (21) eingeführt wird.

10. Maske nach Anspruch 1, **dadurch gekennzeichnet, dass** der geschmeidige hintere Teil (220) des flexiblen Polsters (20) Umfangsrandbereiche (24a-24c) umfasst, die die hintere Atemöffnung (22) umgeben, wobei die Umfangsrandbereiche (24a-24c) eine dünne Umfangswand, beispielsweise eine Membran, aufweisen, die eine Abdichtung um die hintere Öffnung (22) herum bildet, wenn die Maske (1) getragen wird.

11. Maske nach Anspruch 1, **dadurch gekennzeichnet, dass** das Polster (20) aus Silikon besteht.

12. Maske nach Anspruch 1, **dadurch gekennzeichnet, dass** in der hinteren Öffnung (22) ein in das Polster (20) hineinragendes Stützelement (25) angeordnet ist.

13. Maske nach Anspruch 1, **dadurch gekennzeichnet, dass** ein röhrenförmiges Hohlverbindungsstück (51) durch die vordere Öffnung (11) des Maskenkörpers (10) in die Kragenstruktur (17) eingeführt und darin befestigt ist, wobei das röhrenförmige Hohlverbindungsstück (51) vorzugsweise an einem flexiblen Schlauch (50) angeordnet ist.

14. Vorrichtung zur Versorgung eines Patienten (P) mit Gas, umfassend ein medizinisches Beatmungsgerät und eine Nasenmaske (1) gemäß einem der vorstehenden Ansprüche, wobei das medizinische Beatmungsgerät mittels des flexiblen Schlauchs (50), der das röhrenförmige Hohlverbindungsstück (51) umfasst, fluidisch und mechanisch mit der Nasenmaske (1) verbunden ist.

## Revendications

1. Masque nasal respiratoire (1) comprenant :
- un corps de masque (10) comprenant :
. une section avant (110) comprenant une ouverture avant (11) et une surface arrière (112),
. une section arrière tubulaire (120) comprenant un premier passage interne (13) et une ouverture arrière (12), ladite ouverture arrière (12) étant en communication fluidique avec l'ouverture avant (11) via le premier passage interne (13), et comprenant en outre un premier moyen de fixation (130) et
- un coussin flexible creux (20) comprenant :
. une partie avant tubulaire (210) comprenant une ouverture avant (21) et des seconds moyens de fixation (230), et
. une partie arrière souple (220) comprenant une ouverture de respiration arrière (22) pour permettre, en utilisation, des échanges gazeux avec les narines d'un utilisateur (P), ladite ouverture de respiration arrière (22) étant en communication fluidique avec ladite ouverture avant (21), ladite partie arrière souple (220) comprenant des régions de bord périphériques (24) entourant l'ouverture de respiration arrière (22) qui sont configurées pour, lors de l'utilisation, entrer en contact et assurer une étanchéité aux gaz avec des zones situées immédiatement autour des bords des narines de l'utilisateur (P) comprenant une zone de lèvre supérieure (ULA) située immédiatement sous les narines, deux ailes courbes (CA) de part et d'autre des narines et une région de pointe (TR) du nez,
et dans laquelle :
- la surface arrière (112) du corps de masque (10) fait face au coussin flexible (20),
- la partie arrière tubulaire (120) du corps du masque (10) est insérée coaxialement (XX) dans la partie avant tubulaire (210) du coussin souple (20), et
- les premiers moyens de fixation (130) de la section arrière tubulaire (120) du corps de masque (10) coopèrent avec les seconds moyens de fixation (230) de la partie avant tubulaire (210) du coussin souple (20) pour fixer de manière amovible le coussin souple creux (20) directement au corps de masque (10),
**caractérisé en ce que** :
- la surface arrière (112) de la partie avant (110) du corps de masque (10) porte une structure en collerette (17) faisant saillie à partir de ladite surface arrière (112) vers le coussin souple (20), et comprenant un deuxième passage interne (18) en communication fluidique avec l'ouverture avant (11) et avec le premier passage interne (13) de la section arrière tubulaire (120), la section arrière tubulaire (120) et la structure en collerette (17) étant disposées coaxialement (XX) et à distance l'une de l'autre, et
- la section avant (110) du corps de masque (10) comprend en outre plusieurs orifices de ventilation (16) traversant la paroi avant (110), disposés autour de l'ouverture avant (11) et s'ouvrant sur la surface arrière (112) du corps de masque (10) dans l'espace entre la structure de collerette (17) et la section arrière tubulaire (120) du corps de masque (10).

2. Masque selon la revendication 1, **caractérisé en ce que** la partie avant (110) du corps du masque (10) comprend en outre deux bras latéraux (14) faisant saillie latéralement de chaque côté dudit corps du masque (10), de préférence les deux bras latéraux (14) sont réalisés d'un seul tenant avec le corps du masque (10).

3. Masque selon la revendication 1, **caractérisé en ce que** :
- les premiers moyens de fixation (130) du corps de masque (10) comprennent un épaulement annulaire (131) faisant saillie vers l'extérieur, disposé sur la partie arrière tubulaire (120) du corps de masque (10),
- le deuxième moyen de fixation (230) du coussin souple (20) comprend une rainure annulaire (231) disposée dans la paroi périphérique intérieure (211) de la partie avant tubulaire (210) du coussin souple (20), et
- l'épaulement annulaire (131) du corps de masque (10) est logé et retenu dans la rainure annulaire (231) du coussin souple (20), lorsque le coussin souple creux (20) est fixé au corps de masque (10).

4. Masque selon la revendication 3, **caractérisé en ce que** la rainure annulaire (231) est délimitée latéralement par une première paroi latérale (232) et une deuxième paroi latérale (233) se faisant face, et lorsque le coussin flexible creux (20) est fixé au corps de masque (10), l'épaulement annulaire (131) du corps de masque (10) est logé et retenu dans la rainure annulaire (231) du coussin souple (20) de telle sorte que l'épaulement annulaire (131) est prise en sandwich entre les première et deuxième parois latérales (232, 233), lesdites première et deuxième parois latérales (232, 233) constituant des butées qui limitent ou bloquent la course ou le mouvement de l'épaulement annulaire (131) dans la rainure annulaire (231) du coussin flexible (20).

5. Masque selon la revendication 1, **caractérisé en ce que** la section arrière tubulaire (120), la structure de collerette (17) et la section avant (110) du corps de masque (10) sont moulées d'un seul tenant.

6. Masque selon la revendication 1, **caractérisé en ce que** la rainure annulaire (231) du coussin flexible (20) est délimitée latéralement par une première paroi latérale (232) et une deuxième paroi latérale (232) se faisant face, l'épaulement annulaire (131) étant prise en sandwich entre ladite première paroi latérale (232) et ladite deuxième paroi latérale (232), lorsque ledit épaulement annulaire (131) est logé dans la rainure annulaire (231) du coussin flexible (20).

7. Masque selon la revendication 1, **caractérisé en ce que** le coussin flexible creux (20) comprend une chambre de respiration interne (23), l'ouverture avant (21) et l'ouverture de respiration arrière (22) du coussin flexible (20) étant en communication fluidique avec ladite chambre de respiration interne (23).

8. Masque selon la revendication 1, **caractérisé en ce que** la structure de collerette (17) est configurée pour fixer un connecteur tubulaire creux (51) inséré dans le deuxième passage interne (18) à travers l'ouverture avant (11) de la section avant (110).

9. Masque selon la revendication 1, **caractérisé en ce que** :
- la section arrière tubulaire (120) du corps du masque (10) présente un contour ovale (121) et
- l'ouverture avant (21) du coussin (20) a une forme ovale (212) qui correspond au contour ovale (121) de la section arrière tubulaire (120) du corps du masque (10), lorsque la section arrière tubulaire (120) du corps du masque (10) est insérée dans la partie avant tubulaire (210) du coussin (20), à travers l'ouverture avant (21) du coussin (21).

10. Masque selon la revendication 1, **caractérisé en ce que** la partie arrière souple (220) du coussin flexible (20) comprend des régions de bord périphériques (24a-24c) entourant l'ouverture de respiration arrière (22), lesdites régions de bord périphériques (24a-24c) comprennent une paroi mince périphérique, telle qu'une membrane, formant un joint d'étanchéité autour de ladite ouverture arrière (22), lorsque le masque (1) est porté.

11. Masque selon la revendication 1, **caractérisé en ce que** le coussin (20) est en silicone.

12. Masque selon la revendication 1, **caractérisé en ce qu'**un élément de support (25) faisant saillie dans le coussin (20) est disposé dans l'ouverture arrière (22).

13. Masque selon la revendication 1, **caractérisé en ce qu'**un raccord tubulaire creux (51) est inséré dans la structure de collerette (17) à travers l'ouverture avant (11) du corps de masque (10) et y est fixé, de préférence le raccord tubulaire creux (51) est disposé sur un tuyau flexible (50).

14. Installation pour fournir du gaz à un patient (P) comprenant un ventilateur médical et un masque nasal respiratoire (1) selon l'une quelconque des revendications précédentes, ledit ventilateur médical étant relié de manière fluide et mécanique au masque nasal respiratoire (1) au moyen du tuyau flexible (50) comprenant le connecteur tubulaire creux (51).
